# EUROPEAN PATENT SPECIFICATION

(11) **EP 1 347 057 B1**
(45) Date of publication and mention of the grant of the patent: **20.08.2008**
(21) Application number: 02253703.9
(22) Date of filing: 27.05.2002
(51) Int. Cl.: C12P 13/08, C12N 15/52, C12N 9/88

(54) **Method for L-threonine production**
Verfahren zur Produktion von L-Threonin
Méthode pour la production de L-Threonine

(30) Priority: 21.03.2002 KR 2002015380
(43) Date of publication of application: 24.09.2003
(73) Proprietor: CJ CheilJedang Corporation, Jung-gu Seoul 100-802 (KR)
(72) Inventor: Park, Jae-yong, Gwanak-gu, Seoul (KR); Kim, Dae-cheol, Majang-yeon, Yicheon-city, Kyungki-do (KR); Lee, Byoung-choon, Seongbuk-gu, Seoul (KR); Lee, Jin-ho, Yichoen-city, Kyungki-do (KR); Cho, Jae-yong, Sanghyun-dong, Yongin-city, Kyungki-do (KR); Park, Young-hoon, Seongnam-city, Kyungki-do (KR)
(74) Representative: Gillard, Richard Edward

(56) References cited:
- US-A- 5 538 873
- US-A- 5 939 307
- SCHWEIZER H P ET AL: "Genetic analysis of the tdcABC operon of Escherichia coli K-12." JOURNAL OF BACTERIOLOGY. UNITED STATES NOV 1988, vol. 170, no. 11, November 1988 (1988-11), pages 5360-5363, XP009014030 ISSN: 0021-9193
- GANDURI YOJANA L ET AL: "TdcA, a transcriptional activator of the tdcABC operon of Escherichia coli, is a member of the LysR family of proteins." MOLECULAR & GENERAL GENETICS, vol. 240, no. 3, 1993, pages 395-402, XP001153641 ISSN: 0026-8925
- GUELDENER U ET AL: "A new efficient gene disruption cassette for repeated use in budding yeast" NUCLEIC ACIDS RESEARCH, OXFORD UNIVERSITY PRESS, SURREY, GB, vol. 24, no. 13, 1996, pages 2519-2524, XP002147414 ISSN: 0305-1048

### SEQUENCE LISTING

<110> Cheil Jedang Corporation
<120> Method for L-threonine Production
<130> REG/G19038EP
<140> 02253703.9
   <141> 2002-05-27
<160> 2
<170> PatentIn version 3.1
<210> 1
   <211> 30
   <212> DNA
   <213> Artificial
<220>
   <223> primer
<400> 1
   aggaggggat ccggtatgtc ttctgaggcg 30
<210> 2
   <211> 29
   <212> DNA
   <213> Artificial
<220>
   <223> primer
<400> 2
   aggagggaat tcatcggcaa caggcacag 29

## Description

### BACKGROUND OF THE INVENTION

### 1. Field of the Invention

The present invention relates to the production of L-threonine involving microorganisms. More particularly, the present invention relates to a process for producing L-threonine with a high yield using a microorganism, in which the threonine dehydratase (*tdc*) gene of the genomic DNA of the microorganism is partially deactivated using a recombination technique to thereby markedly increase the yield of L-threonine.

### 2. Description of the Related Art

L-threonine, a kind of essential amino acid, is widely used as an additive to animal feed and food, and as fluids and synthetic materials for medical and pharmaceutical use. L-threonine is produced by fermentation using synthetic mutants derived from wild types of *Escherichia Coli*, *Corynebacterium*, *Serratia*, and *Providencia*. These mutants are known to include amino acid analogs- and pharmaceutical-resistant mutants and synthetic mutants thereof rendered auxotrophic for diaminopimelic acid, methionine, lysine, or isoleucine (Japanese Laid-open Patent Application No. hei 2-219582, Appl., Microbiolo. Biotechnol., 29, 550-553 (1988), and Korean Patent Publication No. 92-8365). Korean Patent Application No. 90-22965 discloses the L-threonine-producing strain TF4076 (KFCC10718) that is auxotrophic for methionine and resistant to threonine analogs (AHV: α-amino-β-hydroxyvaleric acid), lysine analogs (AEC: S-(2-aminoethyl)-L-cysteine), isoleucine analogs (α-aminobutyric acid), and methionine analogs (ethionine).

A common approach to increase the level of expression of a particular gene uses a plasmid that gives a greater copy number to a microorganism in order to increase the number of genes in the microorganism (Sambrook et al., Molecular cloning, Second Edition, 1989, 1.3-1.5). A target gene is integrated into a plasmid, and the host microorganism is transformed with the recombinant plasmid to cause an increase in the number of genes in the host microorganism according to the copy number of the plasmid. A partial success in this type of approach to improve threonine productivity is reported in U.S. Patent No. 5, 538,873. However, most technologies using such recombinant plasmids overexpress a particular gene, which is undesirable for the host microorganism, and causes a problem of plasmid instability so that the plasmid may be lost during cultivation of the recombinant strain.

To address this problem, approaches to add antibiotics to culture media or to use an expression regulatory plasmid were suggested (Sambrook et al. Molecular cloning, Second Edition, 1989, 1.5-1.6 & 1.9-1.11). In the approach of using the expression regulatory plasmid to yield a particular product, cell cultivation is performed under non-expression conditions in the growth stage to reduce disadvantages to the host microorganism and transient expression is induced after full growth of the microorganism. However, most of these expression regulatory plasmids may be used only in the case that the final product is protein.

Producing primary metabolites is closely associated with the growth of microorganisms, so it is difficult to increase the yield of the primary metabolites unless target genes are expressed in the growth stage. The production of threonine, a primary metabolite, is such a case.

As an effort to compensate for this drawback, a particular threonine biosynthetic gene was incorporated into a chromosomal DNA to produce threonine (U.S. Patent No. 5,939,307). However, this approach replaces a chromosomal gene by an inducible promoter-substituted gene, which is hardly expected to markedly increase the expression of the threonine operon gene.

Therefore, the present inventors completed the present invention by inactivating threonine dehydratase (*tdc*) gene in the chromosome, which involves in one of the threonine degradation pathways, to inhibit the degradation of threonine while the other inherent generic function of the host microorganism remains and thus increase the yield of threonine.

Most current genetic engineering techniques applied to increase the yield of threonine are focused on the biosynthetic pathway starting with oxaloacetate. However, the present invention involves also the activity of threonine dehydratase (*tdc*) gene in a threonine degradation pathway to efficiently and markedly increase L-threonine yield.

H P Schweizer et al in "Genetic analysis of the tdcABC operon of Escherichia coli K-12" Journal of Bacteriology, Nov. 1998, p 5360-5363 describes the mapping of the biodegradative threonine dehydratase (*tdc*) operon.

Y L Ganduri in "TdcA, a transcriptional activator of the tdcABC operon of Escherichia coli, is a member of the LysR family of proteins" Mol Gen Genet (1993) 240:395-402 discloses the relationship between *tdc*A and the LysR family of proteins based on its role in *tdc* expression. Plasmids having a deletion in the *tdc* operon are disclosed.

### SUMMARY OF THE INVENTION

To solve the above-described problems, it is an object of the present invention to provide a high yield L-threonine production method which eliminates problems of plasmid instability and microbial growth inhibition arising with recombinant plasmid-bearing strains and markedly increases L-threonine productivity by suppressing the activity of a threonine dehydratase.

To achieve the object of the present invention, there is provided a recombinant plasmid incorporating a deactivated *tdc* (threonine dehydratase) gene.

The *tdc* operon of a threonine-producing microorganism is deactivated by cleaving a cleavage site present in *tdc* B and cleaving a cleavage site present in *tdc* C of the *tdc* operon and inserting a cassette with an antibiotic marker into the resulting site. The threonine producing microorganism is an *E.coli* strain.

The *E.coli* strain is also resistant to threonine analogs, lysine analogs, isoleucine analogs, and methionine analogs. In an embodiment of the present invention, additional one or more copies of each of the phosphoenolpyruvate carboxylase (*ppc*) gene and the threonine operon are integrated into the chromosomal DNA of the *E.coli* strain. Preferably, the *E.coli* has the name pGmTN-PPC12 of Accession No. KCCM-10236.

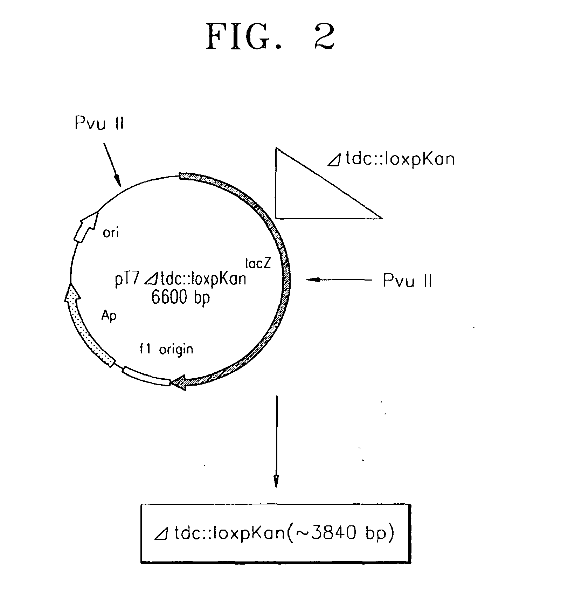

The present invention also provides an *E.coli* strain transformed with the recombinant plasmid described above. A *tdc* B and *tdc* C gene fragment including an antibiotic marker originated from the recombinant plasmid may be inserted into the genome of the *E.coli* strain. Preferably, the *tdc* B and *tdc* C gene fragment including the antibiotic marker is Δ*tdc*::loxpKan of FIG. 2. Most preferably, the *E.coli* strain has the name TRN212 of Accession No. KCCM-10353.

The object of the present invention is achieved by a method for producing L-threonine using the *E.coli* strain described above, preferably, having the name TRN212 of Accession No. KCCM-10353.

The present invention will now be described in greater detail.

There are a few known L-threonine metabolic pathways (Neihardt FC et al. (eds) Escherichia coli and Salmonella: Cellular and Molecular Biology, 2nd edn. ASM Press., Washington D.C., pp 369-370), including three major pathways: a first pathway involving threonine dehydrogenase that catalyzes the decomposition of threonine to α-amino-β-ketobutyrate which is then converted into acetyl-CoA and glycine, or decomposed further to form aminoacetone and converted to pyruvate; a second pathway involving threonine dehydratase which generates α-ketobutyrate to be metabolized to propionyl-CoA and finally to succinyl-CoA, an intermediate of the TCA cycle; and a third pathway involving threonine aldolase.

The present invention is characterized in that the activity of the threonine dehydratase in the L-threonine metabolic pathway is suppressed to cause maximum accumulation of L-threonine.

In the L-threonine production method according to the present invention, pGmTN-PPC12 (KCCM-10236, Korean Patent Application No. 01-6976 filed by the applicant) derived from the *E.coli* TF4076 (KFCC10718, Korean Patent Application No. 90-22965) was used as a L-threonine producing strain. The pGmTN-PPC12 strain (KCCM-10236) is obtained by inserting a phosphoenolpyruvate carboxylase (*ppc*) gene from the chromosome of the L-threonine producing *E.coli* strain, TF4076 (KFCC 10718), by polymerase chain reaction (PCR) and a threonine operon cloned from the same chromosome into the chromosome of the host *E.coli* strain TF4076, so that there are twice as many *ppc* gene and threonine operon in the chromosomal DNA as there are in the TF4076 stain. The pGmTN-PPC12 strain can enhance the expression of the *ppc* gene involved in the conversion of phosphoenolpyruvate (PEP) to a threonine biosynthesis precursor, oxaloacetete, and the genes encoding enzymes involved in the synthetic pathway of threonine from oxaloacetate, including *thr*A (aspartokinase I-homoserine dehydrogenase), *thr*B (homoserine kinase), and *thr*C (threonine synthase), and thus can improve L-threonine productivity. The pGmTN-PPC12 strain was deposited January 5, 2001 with the Korean Collection for Type Cultures (KCTC) and was given Accession Number KCCM 10236.

In the present invention, the threonine dehydratase (*tdc*) gene, which engages in a threonine metabolic pathway of pGmTN-PPC12 (KCCM-10236), is deactivated, to thus increase the yield of L-threonine.

Threonine dehydratase is known to be an operon to express under low-level of oxygen and high-level of threonine concentration conditions. The expression of the *tdc* operon is also induced at low-glucose content as in the latter half of fermentation. Therefore, it is necessary to deactivate the threonine dehydratase to discover high-throughput threonine producing strains.

### BRIEF DESCRIPTION OF THE DRAWINGS

The above object and advantages of the present invention will become more apparent by describing in detail preferred embodiments thereof with reference to the attached drawings in which:
FIG. 1 depicts the process of constructing recombinant plasmid pT7Blue/*tdc*;
FIG. 2 depicts the process of constructing recombinant plasmid pT7Δ*tdc*::loxpKan and DNA fragment Δ*tdc*::loxpKan; and
FIG. 3 shows the results of a Southern blot analysis obtained by image scanning using a FLA-5000 imaging system (FUJIFILM), where Lanes 1 and 4 indicate size markers, and Lanes 2 and 3. indicate TRN212 (KCCM-10353) and host strain pGmTN-PPC12.

### DETAILED DESCRIPTION OF THE INVENTION

The L-threonine producing method according to the present invention will be described in greater detail.

### 1. Construction of Recombinant Plasmid

Chromosomal DNA is isolated from a threonine producing strain. By using the chromosomal DNA as a template, recombinant plasmid pT7Blue/*tdc* including *tdc* B and *tdc* C of the *tdc* operon is obtained. Any cloning vector can be used without limitation, but pT7Blue cloning vector is preferred.

Recombinant plasmid pT7Δ*tdc*::loxpKan containing a defective *tdc* gene is obtained by integrating a kanamycin-resistant gene fragment including *lox* p sites into the recombinant plasmid pT7Blue/*tdc*.

### 2. Integration of Recombinant Plasmid and Screening

*E.coli* is transformed with the recombinant plasmid pT7Δ*tdc*::loxpKan. Plasmid DNA is isolated and digested with a restriction enzyme, and DNA fragments (Δ*tdc*::loxpKan) are isolated from the digest by electrophoresis. This process is illustrated in FIG. 2.

The threonine producing strain is transformed with the resulting DNA fragment Δ*tdc*::loxpKan. This transformation is performed via homologous recombination which allows the replacement of the inherent *tdc* gene region of the *E.coli* strain with the recombinant DNA fragment pT7Δ*tdc*::loxpKan.

The resulting transformant is inoculated on a kanamycin-containing solid medium to collect colonies. Finally, a target recombinant strain containing a deactivated *tdc* gene is obtained.

For the recombinant strain, the *tdc* gene remains deficient despite the proliferation of the strain, with an excellent L-threonine productivity of 20% higher than the host strain.

The recombinant plasmid can be collected from the transformed strain using known methods, for example, using alkali solution (Sambrook et al., Molecular Cloning, Vol. 1, 1.25-1.28). In particular, Solution 1 (50 mM glucose, 25 mM Tris-HCI, and 10 mM EDTA) is added to weaken the cell membrane of the transformant, and Solution 2 (0.2N NaOH, 1% SDS) is added to destruct the cell membrane and denaturate the cellular constituents of protein and chromosome, and Solution 3 (5M potassium acetate, acetic acid) is added to coagulate the constituents except for the recombinant plasmid. The recombinant plasmid fraction is separated by centrifugation, and only the recombinant plasmid is precipitated with an addition of ethanol and recovered.

The present invention will be described with reference to the following examples. The following examples are for illustrative purposes and are not intended to limit the scope of the invention.

### Example 1: Construction of Recombinant Plasmid and knock-out of tdc gene using the Recombinant Plasmid

The genomic DNA of pGmTN-PPC12 (KCCM-10236), a threonine producing strain, was isolated using a QIAGEN Genomic-tip system. A gene fragment of about 3.1 kb of the *tdc* operon (5295 pb) that contains the *tdc* B and *tdc* C was amplified through polymerase chain reaction (PCR) by using the genomic DNA as a template. The primers used were 5'-agg agg gga tcc ggt atg tct tct gag gcg-3' and 5'-agg agg gaa ttc atc ggc aac agg cac ag-3'. The PCR was achieved through 30 cycles of amplification, each cycle including denaturation at 94°C for 30 seconds, annealing at 56°C for 30 seconds, and extension at 72°C for 3 minutes and 30 seconds.

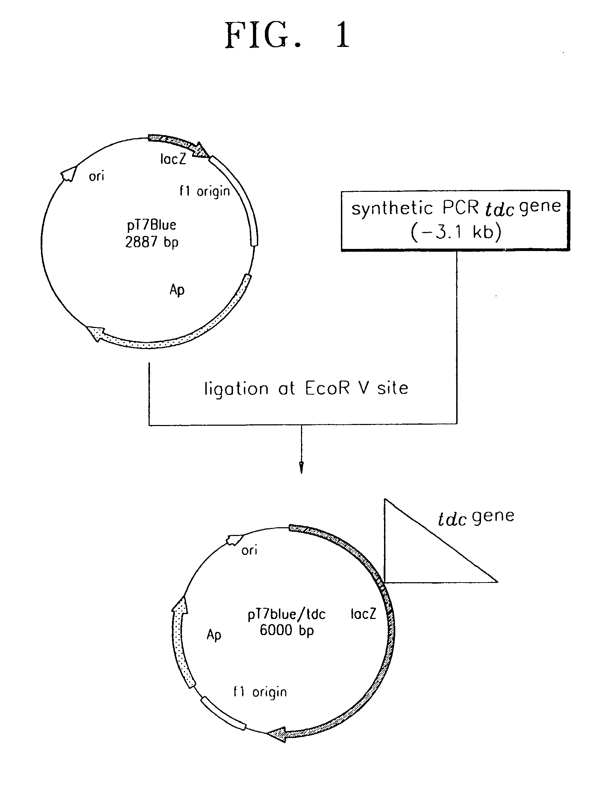

The PCR products were subjected to electrophoresis in 0.7% agarose gels to elute bands of a desired size. The eluted bands were ligated by blunt-end ligation with pT7Blue cloning vector (Novagen Co,) overnight at 16°C to obtain recombinant plasmid pT7Blue/*tdc* (see FIG. 1). *E.coli* DH5α was transformed with the resulting recombinant plasmid pT7Blue/*tdc*, plated on a solid medium containing 50 mg/L of ampicillin, and incubated overnight at 37°C.

Colonies were tooth-picked from the culture, inoculated into a 3-mL liquid medium, and incubated overnight at 200 rpm. Plasmid DNA was isolated from the culture using a QIAGEN mini prep. kit, and the size of the plasmid DNA was identified. The plasmid DNA was digested with restriction enzymes, Sma I and Pst I, and subjected to electrophoresis in 0.7% agarose gels to identify whether its orientation is *tdc* R-A-B-C. The identified plasmid DNA, pT7Blue/*tdc*, was digested with restriction enzymes, Bgl II and Hpa I (of about 1.1 kb each), and the digest was loaded onto 0.7% agarose gels to elute bands of about 4.9 kb. The eluted bands were reacted with the *Klenow* enzyme to generate blunt ends. A kanamycin-resistant gene fragment of about 1.7 kb containing the *lox* p site that obtained by reacting plasmid pUG6 (Ulrich et al., A new efficient gene disruption cassette for repeated use in budding yeast, Nucleic Acids Research, 1996, 24, pp. 2519-2524) with restriction enzymes Hinc II and EcoR V, is ligated to the isolated fragment of pT7Blue/*tdc* by blunt end ligation, thereby resulting recombinant plasmid pT7Δ*tdc*::loxpKan.

### Example 2: Screening of Strain integrated with Recombinant Plasmid

*E.coli* DH5α was transformed with the recombinant plasmid pT7Δ*tdc*::loxpKan, plated on a solid medium containing 50 mg/L of ampicillin and 15 mg/L of kanamycin, and incubated overnight at 37°C. Colonies were picked from the culture, inoculated into a 3-mL liquid medium containing ampicillin and kanamycin, and incubated overnight at 200 rpm. Plasmid DNA was isolated from the culture using a QIAGEN mini prep. kit, and the size of the plasma DNA was identified. The plasmid DNA was digested with a restriction enzyme and loaded onto 0.7% agarose gels to identify its orientation. The identified plasmid DNA was digested with restriction enzyme PVU II and subjected to electrophoresis in 0.7% agarose gels to elute a DNA fragment (Δ*tdc*::loxpKan) of about 3840 bp. pGmTN-PPC12, a threonine producing strain, was transformed with the DNA fragment Δ*tdc*::loxpKan by electroporation and plated on a kanamycin-containing solid medium to screen colonies, giving recombinant strains having the deactivated *tdc* gene.

### Example 3: Comparison of Theonine Productivity in Flask Cultivation for obtained Recombinant Strains

Thirty single colonies of the recombinant strains cultured in the solid medium containing kanamycin in Example 2 were screened for L-threonine productivity comparisons using threonine titer media in Erlenmeyer flasks. The composition of the threonine titer medium used in each case is shown in Table 1.

**Table 1. Composition of Threonine Titer Medium**

| | |
|---|---|
| Component | Amount per liter |
| Glucose | 70 g |
| (NH₄)₂SO₄ | 28 g |
| KH₂PO₄ | 1.0 g |
| MgSO₄·7H₂O | 0.5 g |
| FeSO₄·7H₂O | 5 mg |
| MnSO₄·8H₂O | 5 mg |
| Calcium carbonate | 30 g |
| L-methionine | 0.15 g |
| Yeast extract | 2 g |
| pH 7.0 | |

Single colonies were cultured on LB solid media overnight at 32°C in an incubator. 25 mL of the titer medium was inoculated with a loopful of each culture and incubated at 32°C, 250 rpm for 48 hours. The results of the analysis are shown in Table 2. 28 colonies of thirty colonies of the new recombinant strains show excellent productivity, including eight colonies that produced 26 g/L or greater threonine, compared to the host strain, pGmTN-PPC12, which produced 23 g/L of threonine. The recombinant strain, which recorded the highest threonine productivity at 27 g/L with a higher yield of about 17.4% than the host strain, was observed and named "TRN212". The mutant strain TRN212 was deposited February 19, 2002 with the Korean Collection for Type Cultures (KCTC) and was given Accession Number KCCM-10353.

**Table 2. Results of Flask Titer Test for Recombinant Strains**

| | | | | |
|---|---|---|---|---|
| L-threonine Concentration | 20-23 g/L | 23-24.5 g/L | 24.5-26 g/L | 26 g/L or greater |
| Colony Counts | 2 | 9 | 11 | 8 |

### Example 4: Confirmation of knock-out of tdc gene using Southern Blotting Analysis

Southern blotting was performed to verify the specific knock-out of the *tdc* gene of the recombinant strain obtained in Example 3. The host strain pGmTN-PPC12 and the recombinant strain TRN212 (KCCM-10353) were incubated overnight in a 30 mL liquid medium at 200 rpm, and genomic DNA was isolated using a QIAGEN genomic kit 20. The isolated genomic DNA was digested with restriction enzyme EcoR I and subjected to electrophoresis in 0.7% agarose gels to separate DNA fragments by size. The size-separated DNA fragments were attached to a nylon membrane (YOUNG Sci. Biodyne B Membrane) overnight by capillary transfer (Molecular Cloning, Vol. 1, 6.31-6.38) of the electrophoretic gel, followed by dehydration and UV radiation (120 mJ/cm², SpectroLinker^{™}) to immobilize the DNA fragments on the nylon membrane (Molecular Cloning, Vol. 1, 6.45). The DNA fragments affixed to the nylon membrane was prehybridized in Prehybridization Buffer I (Roche #1093657) at 55°C for 2 hours and then hybridized in a hybridization oven (BAMBINO 230300) with addition of previously prepared denatured DNA probe.

The denatured probe used was prepared as follows. Plasmid pUG6 isolated using a QIAGEN kit was digested with restriction enzymes, Hinc II and EcoR V, to obtain a kanamycin-resistant gene fragment of about 1.7 kb that contained the lox p site. This gene fragment was heated in 100°C boiling water for 5 minutes and cooled using ices for 5 minutes to generate single-stranded DNAs. The single-stranded DNAs were reacted overnight at 37°C using a DIG Labelling and Detection Kit (Roche #1093657) to generate DIG-UDP-integrated DNA probes.

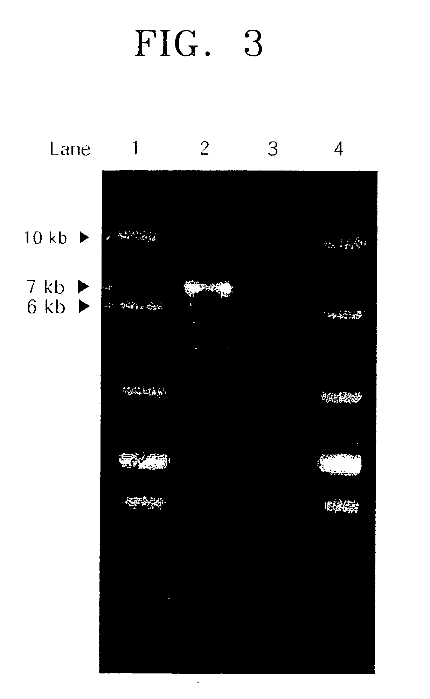

After hybridization, the DNA fragments non-specifically bound to the membrane were removed using Washing Solutions I and II (Roche #1093657). Then, the membrane was treated with Prehybridization Buffer 2 (Roche #1093657) at room temperature for 30 minutes for masking and reacted with an addition of anti-DIG antibody that is specifically coupled to DIG-UTP, at room temperature for 30 minutes. The anti-DIG antibody non-specifically bound to the membrane was removed using Washing Solution III (Roche #1093657), and the membrane was subjected to color reaction using a Labelling and Detection kit (Roche #1093657) until bands appeared. The results are shown in FIG. 3. No band appeared for the host strain pGmTN-PPC12 (Lane 3) without the kanamycin gene. In contrast, a band of about 7 kb was observed in Lane 2 for the new recombinant strain according to the present invention, TRN212 (KCCM-10353), which was expected. A *tdc* gene fragment of about 5.3 kb resulting from the digestion with restriction enzyme EcoR I and the kanamycine-resistant gene of about 1.7 kb amount to about 7.0 kb. Lane 1 and Land 4 represent size markers.

### Example 5: Comparison of L-threonine Productivity using Fermentor

L-threonine productivity in a 5-L fermentor was compared between host strain pGmTN-PPC12 and recombinant strain TRN212 (KCCM-10353) having the deactivated *tdc* gene, which had been screened in Example 2 and undergone the test to confirm the knock-out of the gene in Example 4. The initial medium composition used is shown in Table 3. LB media further containing per liter 10 g of glucose and 0.1 g of L-methionine were used for seed culture, and an initial volume of inoculation into a fermentor was determined at 3-5% by volume of a target initial culture. Glucose was added to a final concentration of 5% by weight each time, over 6 times in total, along with 1% by weight of KH₂PO₄. Here, each addition of glucose was determined by deletion of glucose. The initial volume of the culture was 1.5L and the final volume of the culture was 3.0L. A total concentration of glucose added through fermentation was 250 g/L. During fermentations, the medium was stirred at 700-1000 rpm, temperature was controlled at 32°C, and pH was adjusted at 7.0 with 25-28% ammonia water. Air-flow velocity was adjusted at 0.5 vvm. The results are shown in Table 4. As shown in Table 4, the host strain TRN212 produces 93.5 g/L of L-threonine with a yield of 37.4% with respect to glucose consumption. In contrast, recombinant strain TRN212 produces 112 g/L of L-threonine with a yield of 45.2%, which is 21% higher than the host strain TRN212. In addition, a similar fermentation pattern as the host strain was observed on the recombinant strain, without reduction in sugar consumption during fermentation, which often appears on recombinant strains due to growth inhibition.

**Table 3. Initial Medium Composition in 5-L Fermentor**

| Component | Amount per liter |
|---|---|
| Glucose | 50 g |
| KH₂PO₄ | 4 g |
| (NH₄)₂SO₄ | 6 g |
| Yeast extract | 3 g |
| MgSO₄·7H₂O | 2 g |
| L-methionine | 1 g |
| FeSO₄·7H₂O | 40 mg |
| MnSO₄·8H₂O | 10 mg |
| CaCl₂·2H₂O | 40 mg |
| CoCl₂·6H₂O | 4 mg |
| H₃BO₃ | 5 mg |
| Na₂MoO₄·2H₂O | 2 mg |
| ZnSO₄·7H₂O | 2 mg |
| pH 7.0 | |

**Table 4. Results of Fermentative Production of Threonine by Recombinant Strains**

| Strain | Threonine (g/L) | Fermentation Time (hr) | Yield (%) |
|---|---|---|---|
| PGmTN-PPC12 | 93.5 | 78 | 37.4 |
| TRN212 | 112 | 77 | 45.2 |

The new recombinant strain of the present invention is used in producing L-threonine, both amount and yield are improved greater than 20% compared to the host strain, without reduction in sugar consumption during fermentation, which often appears on recombinant strains due to growth inhibition.

## Claims

1. A recombinant plasmid having a deactivated *tdc* operon of a threonine producing microorganism wherein the *tdc* operon is deactivated by cleaving a cleavage site present in *tdc*B *and cleaving a cleavage site present in tdc*C of the *tdc* operon and inserting a cassette with an antibiotic marker into the resulting site, and wherein the threonine producing microorganism is *E*.*coli* resistant to threonine analogs, lysine analogs, isoleucine analogs, and methionine analogs.

2. The recombinant plasmid of claim 1, wherein the recombinant plasmid is constructed by deactivation of the *tdc* gene of pT7Blue/*tdc*, wherein pT7Blue/*tdc* is obtainable by amplifying a gene fragment of about 3.1 kb of the *tdc*operon that contains the *tdc*B and *tdc*C by PCR using oligonucleotides 5'-agg agg gga tcc ggt atg tct tct gag gcg-3' and 5'-agg agg gaa ttc atc ggc aac agg cac ag-3' as primer and genomic DNA of *E.coli* having accession no. KCCM-10236 as template; and
blunt end ligating the gene fragment with pT7Blue vector cleaved with restriction enzyme EcoRV.

3. The recombinant plasmid of claim 1 expressed as pT7Δ*tdc*::loxpKan, wherein pT7Δ*tdc*::loxpKan is obtainable by constructing pT7Blue/*tdc* according to claim 2 and obtaining a blunt end fragment of about 4.9 kb by cleaving the plasmid pT7Blue/*tdc* with Bgl II and Hpal, eluting the fragment of about 4.9 kb and reacting with Klenow enzyme; and
ligating the 4.9 kb blunt end fragment of pT7Blue/*tdc* with a kanamycin resistant gene fragment of about 1.7 kb containing the lox p site that obtained by reacting the plasmid pUG6 with Hincll and EcoRV.

4. An *E.coli* strain transformed with the recombinant plasmid of any of claims 1 to 3.

5. The *E.coli* strain of claim 4, wherein a *tdc*B and *tdc*C gene fragment including an antibiotic marker originated from the recombinant plasmid is integrated into the genome of the *E.coli* strain.

6. The *E*.*coli* strain of claim 5 having the name TRN212 of accession no. KCCM-10353.

7. The *E*.*coli* strain of claim 4, wherein the *E.coli* strain is obtainable by transforming an *E*.*coli* having additional one or more copies of each of the phosphoenolpyruvate carboxylase (ppc) gene and the threonine operon integrated into the chromosomal DNA thereof with the recombinant plasmid of any one of claims 1 to 3.

8. The *E.coli* strain of claim 7, wherein the *E*.*coli* strain is obtainable by transforming an *E.coli* having accession no. KCCM-10236 with the recombinant plasmid of any one of claims 1 to 3.

9. A method for producing L-threonine using the *E.coli* strain according to any of claims 4 to 8.

## Patentansprüche

1. Rekombinantes Plasmid mit einem desaktivierten *tdc*-Operon eines Threonin produzierenden Mikroorganismus, wobei das *tdc*-Operon durch Spalten einer Spaltungsstelle, die in *tdc*B vorliegt, und Spalten einer Spaltungsstelle, die in *tdc*C des *tdc*-Operons vorliegt, und Insertieren einer Kassette mit einem Antibiotika-Marker in die erhaltene Stelle desaktiviert wird, und wobei der Threonin produzierende Mikroorganismus *E*. *coli* ist, der gegen Threoninanaloge, Lysinanaloge, Isoleucinanaloge und Methioninanaloge resistent ist.

2. Rekombinantes Plasmid nach Anspruch 1, wobei das rekombinante Plasmid konstruiert ist durch Desaktivierung des *tdc*-Gens von pT7Blue/*tdc*, wobei pT7Blue/*tdc* durch Amplifizieren eines Genfragments von ungefähr 3,1 kb des *tdc*-Operons, das *tdc*B und *tdc*C enthält, mit PCR unter Verwendung von Oligonukleotiden 5'-agg agg gga tcc ggt atg tct tct gag gcg-3' und 5'-agg agg gaa ttc atc ggc aac agg cac ag-3' als Primer und genomischer DNA von *E*. *coli* mit der Zugangsnummer KCCM-10236 als Templat erhältlich ist, und
Blunt-End-Ligation des Genfragments mit einem pT7Blue-Vektor, der mit Restriktionsenzym EcoRV gespalten ist.

3. Rekombinantes Plasmid nach Anspruch 1, exprimiert als pT7Δ*tdc*::IoxpKan, wobei pT7Δ*tdc*::loxpKan erhältlich ist durch Konstruieren von pT7Blue/*tdc* nach Anspruch 2 und Erhalten eines Blunt-End-Fragments von ungefähr 4,9 kb durch Spalten des Plasmids pT7Blue/*tdc* mit Bgl II und Hpal, Eluieren des Fragments von ungefähr 4,9 kb und Umsetzen mit Klenow-Enzym, und
Ligation des Blunt-End-Fragments von 4,9 kb von pT7Blue/*tdc* mit einem gegen Kanamycin resistenten Genfragment von ungefähr 1,7 kb, das die lox p-Stelle enthält, erhalten durch Umsetzen des Plasmids pUG6 mit Hinc II und EcoRV.

4. Stamm von *E*. *coli*, der mit dem rekombinanten Plasmid nach einem der Ansprüche 1 bis 3 transformiert ist.

5. Stamm von *E*. *coli* nach Anspruch 4, wobei ein *tdc*B- und *tdc*C-Genfragment mit einem Antibiotika-Marker, das vom rekombinanten Plasmid stammt, in das Genom des Stamms von *E*. *coli* integriert ist.

6. Stamm von *E*. *coli* nach Anspruch 5 mit der Bezeichnung TRN212 der Zugangsnummer KCCM-1 0353.

7. Stamm von *E*. *coli* nach Anspruch 4, wobei der Stamm von *E*. *coli* durch Transformieren eines *E*. *coli*, der zusätzlich eine oder mehrere Kopien des Phosphoenolpyruvatcarboxylase(ppc)-Gens und des Threoninoperons integriert in seine chromosomale DNA aufweist, mit dem rekombinanten Plasmid nach einem der Ansprüche 1 bis 3 erhältlich ist.

8. Stamm von *E*. *coli* nach Anspruch 7, wobei der Stamm von *E*. *coli* durch Transformieren eines *E*. *coli* mit der Zugangsnummer KCCM-10236 mit dem rekombinanten Plasmid nach einem der Ansprüche 1 bis 3 erhältlich ist.

9. Verfahren zur Herstellung von L-Threonin unter Verwendung des Stamms von *E*. *coli* nach einem der Ansprüche 4 bis 8.

## Revendications

1. Plasmide recombiné comprenant un opéron *tdc* désactivé d'un microorganisme produisant de la thréonine dans lequel l'opéron *tdc* est désactivé en clivant un site de reconnaissance se trouvant dans *tdc*B et en clivant un site de reconnaissance se trouvant dans *tdc*C de l'opéron *tdc* puis en insérant une cassette comprenant un marqueur antibiotique dans le site ainsi obtenu, et dans lequel le microorganisme produisant de la thréonine est *E*. *coli* résistant aux analogues de la thréonine, aux analogues de la lysine, aux analogues de l'isoleucine et aux analogues de la méthionine.

2. Plasmide recombiné selon la revendication 1, dans lequel le plasmide recombiné est construit par désactivation du gène *tdc* de pT7BIue/*tdc*, dans lequel pT7BIue/*tdc* peut être obtenu par amplification d'un fragment de gène d'environ 3,1 kb de l'opéron *tdc* qui contient le *tdc*B et le *tdc*C par PCR en utilisant des oligonucléotides 5'-agg agg gga tcc ggt atg tct tct gag gcg-3' et 5'-agg agg gaa ttc atc ggc aac agg cac ag-3' en tant qu'amorce et l'ADN génomique d'*E*. *coli* portant le numéro d'enregistrement KCCM-10236 en tant que matrice ; et
en ligaturant les extrémités franches du fragment de gène dans le vecteur pT7Blue clivé avec l'enzyme de restriction EcoRV.

3. Plasmide recombiné selon la revendication 1 exprimé en tant que pT7Δ*tdc*::loxpKan, dans lequel pT7Δ*tdc*::loxpKan est obtenu en construisant pT7Blue/*tdc* selon la revendication 2, puis en obtenant un fragment à extrémités franches d'environ 4,9 kb par clivage du plasmide pT7Blue/*tdc* avec Bgl II et Hpal, en éluant le fragment d'environ 4,9 kb et en le faisant réagir avec une enzyme de Klenow ; et
en ligaturant le fragment à extrémités franches de 4,9 kb de pT7Blue/*tdc* avec un fragment de gène résistant à la kanamycine d'environ 1,7 kb contenant le site lox p obtenu en faisant réagir le plasmide pUG6 avec HincII et EcoRV.

4. Souche d'*E*. *coli* transformée avec le plasmide recombiné selon l'une quelconque des revendications 1 à 3.

5. Souche d'*E*. *coli* selon la revendication 4, dans laquelle un fragment de gène *tdc*B et *tdc*C comprenant un marqueur antibiotique provenant du plasmide recombiné est intégré dans le génome de la souche d'*E*. *coli.*

6. Souche d'*E*. *coli* selon la revendication 5 portant le nom TRN212 et le numéro d'enregistrement KCCM-10353.

7. Souche d'E. *coli* selon la revendication 4, dans laquelle la souche d'*E*. *coli* est obtenue en transformant un *E*. *coli* comprenant une ou plusieurs copies supplémentaires de chacun des gènes phosphoénolpyruvatecarboxylase (ppc) et l'opéron thréonine intégré à l'ADN chromosomique de celui-ci avec le plasmide recombiné selon l'une quelconque des revendications 1 à 3.

8. Souche d'*E*. *coli* selon la revendication 7, dans laquelle la souche d'*E*. *coli* peut être obtenue en transformant un *E*. *coli* portant le numéro d'enregistrement KCCM-10236 avec le plasmide recombiné selon l'une quelconque des revendications 1 à 3.

9. Procédé de production de L-thréonine utilisant la souche d'*E*. *coli* selon l'une quelconque des revendications 4 à 8.
